# EUROPÄISCHE PATENTANMELDUNG

(11) **EP 2 221 001 A1**
(43) Veröffentlichungstag der Anmeldung: **25.08.2010**
(21) Anmeldenummer: 09002281.5
(22) Anmeldetag: 18.02.2009
(51) Int. Cl.: A61B 5/151

(54) **Analysehandgerät**

(71) Anmelder: Roche Diagnostics GmbH, 68305 Mannheim (DE); F.HOFFMANN-LA ROCHE AG, 4070 Basel (CH)
(72) Erfinder: Rödel, Wolfgang, 69123 Heidelberg (DE); Miltner, Karl, 67227 Frankenthal (DE); Baeter, Thorsten, 67136 Fussgoenheim (DE); Frisch, Gerhard, 68535 Edingen-Neckarhausen (DE); Liedtke, Sebastian, 69117 Heidelberg (DE); Heck, Wolfgang, 67227 Frankenthal (DE)
(74) Vertreter: Twelmeier Mommer & Partner

(57) **Zusammenfassung**

Die Erfindung betrifft ein Analysehandgerät mit einer mechanischen Baugruppe, die eine Transporteinrichtung (9) für analytische Verbrauchselemente (4, 5) umfasst, einer Anzeigeeinrichtung (7), einem Sensor (6) zur Untersuchung einer durch einen Lanzettenstich gewonnenen Körperflüssigkeitsprobe, einem Speicher zum Speichern einer Serie von Messergebnissen, und Betätigungselementen (13, 15, 16, 17) zum Bedienen des Analysehandgeräts (1). Das erfindungsgemäße Gerät hat ein erstes Betätigungselement (13), durch dessen Betätigung das Gerät (1) in einen ersten aktiven Zustand versetzt, die mechanische Baugruppe aktiviert und eine Messung vorbereitet wird, wobei in dem ersten aktiven Zustand durch Betätigen eines der Betätigungselemente (13, 15, 16, 17) eine Messung gestartet wird, und ein zweites Betätigungselement (15), durch dessen Betätigung das Gerät (1) in einen zweiten aktiven Zustand versetzt und die Anzeigeeinrichtung (7) ohne aktivieren der mechanischen Baugruppe eingeschaltet wird.

## Beschreibung

Die Erfindung geht aus von einem Analysehandgerät mit den im Oberbegriff des Anspruchs 1 angegebenen Merkmalen.

Derartige Analysehandgeräte werden insbesondere von Diabetikern benötigt, die mehrmals täglich ihren Blutzuckerspiegel überprüfen müssen und dazu eine Körperflüssigkeitsprobe, in der Regel Blut und/oder interstitielle Flüssigkeit, benötigen, die aus einer kleinen Stichwunde gewonnen wird. Mit einem in dem Analysehandgerät enthaltenen Sensor kann eine Analytkonzentration, beispielsweise die Glucosekonzentration, einer durch einen Lanzettenstich gewonnenen Körperflüssigkeitsprobe gemessen werden.

Ständiges Ziel bei der Entwicklung von Analysehandgeräten ist es, Benutzern eine möglichst einfache Handhabung und eine schnelle Durchführung einer Messung zu ermöglichen.

Aufgabe der Erfindung ist es deshalb, einen Weg aufzuzeigen, wie der Benutzerkomfort bei einem Analysehandgerät der eingangs genannten Art verbessert werden kann.

Diese Aufgabe wird durch ein Analysehandgerät mit den im Anspruch 1 angegebenen Merkmalen gelöst. Vorteilhafte Weiterbildungen der Erfindung sind Gegenstand von Unteransprüchen.

Ein erfindungsgemäßes Analysehandgerät kann mit einem ersten Betätigungselement in einen ersten aktiven Zustand versetzt werden, in dem eine mechanische Baugruppe mit einer Transporteinrichtung für analytische Verbrauchselemente, beispielsweise Testelemente und/oder Lanzetten, aktiviert und eine Messung vorbereitet wird. In dem ersten aktiven Zustand kann dann durch Betätigen eines der Betätigungselemente des Analysehandgeräts eine Messung gestartet werden. Ein erfindungsgemäßes Analysehandgerät hat zusätzlich einen zweiten aktiven Zustand, in dem die Anzeigeeinrichtung ohne Aktivieren der mechanischen Baugruppe eingeschaltet wird.

Beim Aktiveren der mechanischen Baugruppe wird mindestens ein Bauteil der Baugruppe bewegt, beispielsweise ein Testelement und/oder eine Lanzette aus einem Vorrat entnommen. Bei einfachen Analysehandgeräten muss die dafür erforderliche Kraft vom Benutzer aufgebracht werden, bei komplexeren Analysehandgeräten, deren mechanische Baugruppe einen Elektromotor enthält, wird ein elektrischer Energiespeicher, in der Regel eine Batterie, belastet. Vorteilhaft muss dieser Aufwand bei einem erfindungsgemäßen Gerät nur dann erfolgen, wenn ein Benutzer auch tatsächlich eine Messung durchführen möchte. Wenn ein Benutzer aber keine Messung durchführen möchte, sondern beispielsweise lediglich eine Serie von früheren Messungen betrachten oder eine Geräteeinstellung ändern, beispielsweise eine in das Gerät integrierte Uhr stellen möchte, kann der mit dem Vorbereiten einer Messung verbundene Aufwand vorteilhaft unterbleiben, insbesondere kann eine Bewegung mechanischer Komponenten unterbleiben.

Ein erfindungsgemäßes Analysehandgerät kann als ein reines Messgerät ausgebildet sein, das zusammen mit einem separaten Stechgerät ein Messsystem bildet. Bevorzugt ist in ein erfindungsgemäßes Analysehandgerät aber ein Stechgerät integriert. In einem solchen Fall enthält die mechanische Baugruppe zusätzlich auch einen Stechantrieb, um Lanzetten in eine Stechbewegung zu versetzen.

Die Verbrauchselemente können beispielsweise Testelemente mit Nachweisreagenzien zur Konzentrationsbestimmung, insbesondere zur fotometrischen oder elektrochemischen Konzentrationsbestimmung, oder Lanzetten sein. Die Verbrauchselemente können insbesondere auch Lanzetten mit integrierten Testelementen sein. Beispielweise können als Testelemente Lanzetten verwendet werden, die einen Testbereich aufweisen. Ein solcher Testbereich kann beispielsweise eine Küvette zur reagenzfreien Untersuchung oder ein Testfeld mit Nachweisreagenzien sein. Testfelder mit Nachweisreagenzien zur fotometrischen oder elektrochemischen Konzentrationsbestimmung werden bei handelsüblichen Teststreifen zur Glukosekonzentrationsbestimmung seit Jahrzehnten verwendet. Entsprechende Testfelder können auf eine Lanzette aufgeklebt sein. Eine solche Lanzette kann einen Kanal, beispielsweise in Form einer Rinne, aufweisen, der durch Kapillarkräfte einen Flüssigkeitstransport zu einem Testfeld bewirkt. Möglich ist es aber auch, in einem erfindungsgemäßen Analysehandgerät als Verbrauchselemente Lanzetten und separate Testelemente zu verwenden. Derartige Testelemente können eine Körperflüssigkeitsprobe von einer Lanzette aufnehmen, die zu diesem Zweck von der mechanischen Baugruppe des Analysehandgeräts zu dem Testelement geführt wird. Möglich ist es auch, dass separate Testelemente eine Körperflüssigkeitsprobe direkt von einer zuvor erzeugten Stichwunde aufnehmen.

Der Verbrauchselementevorrat für ein erfindungsgemäßes Analysehandgerät kann als ein in das Gerät einsetzbares Magazin ausgebildet sein. Ein derartiges Magazin kann Testelemente und/oder Lanzetten in separaten Magazinkammern enthalten. Möglich ist es auch, ein solches Magazin als eine Kassette mit einem aufgewickelten Trägerband auszubilden, das Testelemente und/oder Lanzetten oder Lanzetten mit integrierten Testelementen trägt. Bei einem Analysehandgerät das ein Messgerät und ein Stechgerät ist, wird vorzugsweise ein interner Vorrat von Lanzetten und Stechelementen verwendet. Möglich ist es aber beispielsweise auch, in dem Gerät nur einen Lanzettenvorrat vorzusehen und Testelemente nach einem Stich für eine Messung einzeln in das Gerät einzuführen.

Eine Messung kann beispielsweise vorbereitet werden, indem ein Testelement und/oder eine Lanzette aus einem Vorrat entnommen werden, indem ein mechanischer Energiespeicher eines Stechantriebs aufgeladen wird, oder indem ein Stechantrieb an eine Lanzette ankoppelt. Bei einem erfindungsgemäßen Gerät können zur Vorbereitung einer Messung alle diese Schritte oder nur ein Teil dieser Schritte durchgeführt werden. Beispielsweise ist es auch möglich, dass ein Testelement oder eine Lanzette nach Abschluss einer Messung aus einem Vorrat entnommen und in eine Gebrauchsposition gebracht werden, in der das Testelement bzw. die Lanzette bis zur nächsten Messung bleiben. Bevorzugt werden Testelemente und/oder Lanzetten aber erst bei der Vorbereitung einer Messung im ersten aktiven Zustand des Geräts aus einem Magazin entnommen, da diese in dem Magazin am besten vor schädlichen Umwelteinflüssen geschützt sind.

Weitere Einzelheiten und Vorteile der Erfindung werden anhand eines Ausführungsbeispiels unter Bezugnahme auf die beigefügten Zeichnungen erläutert. Es zeigen:
- Figur 1: eine schematische Darstellung eines tragbaren Blutzuckermessgeräts; und
- Figur 2: eine schematische Darstellung des in Figur 1 dargestellten Messgeräts mit aufgeschnittenem Gehäuse.

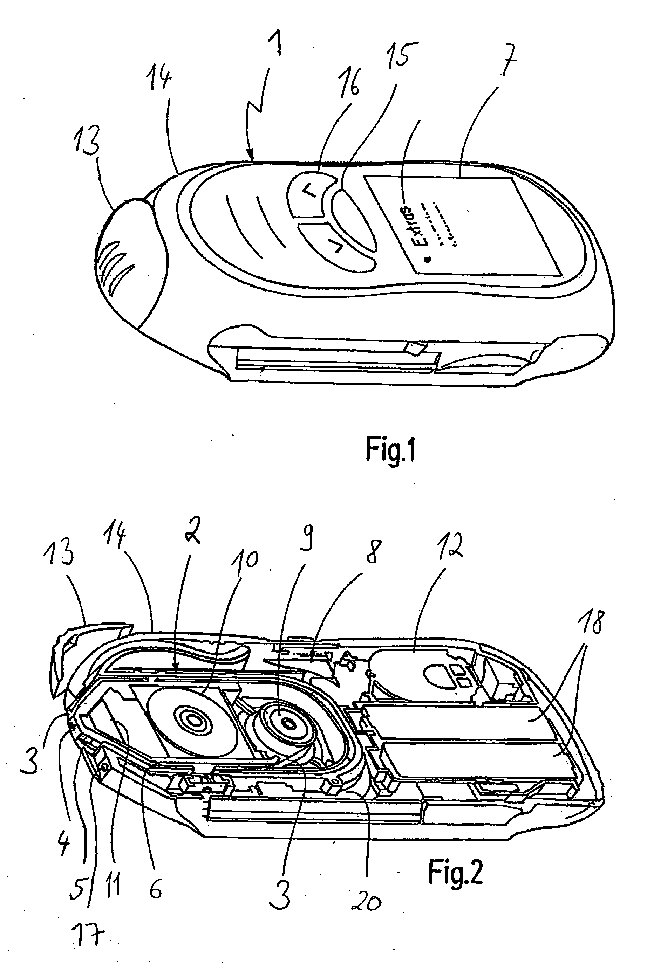

Das in den Figuren 1 und 2 dargestellte Analysehandgerät 1 ermöglicht einem Benutzer die Bestimmung der Glukosekonzentration einer Körperflüssigkeitsprobe in einem weitgehend automatischen Messablauf. In ein Aufnahmefach des Geräts 1 ist ein Magazin 2 mit einem Testelementvorrat einsetzbar. Hierfür ist ein bodenseitiger Gehäusedeckel 20 abnehmbar. Bei dem dargestellten Ausführungsbeispiel ist das Magazin 2 eine Bandkassette, die auf einem Trägerband 3 Lanzetten 4 und Testelemente 5 trägt. Anstelle einer solchen Kassette kann beispielsweise auch ein Magazin mit einzelnen Magazinkammern verwendet werden.

Eine durch einen Lanzettenstich gewonnene Körperflüssigkeitsprobe kann mit einem Sensor 6 untersucht und eine Analytkonzentration, beispielsweise die Glucosekonzentration oder die Lactatkonzentration, bestimmt werden. Messergebnisse können mit einer Anzeigeeinrichtung 7 des Geräts 1, beispielsweise einer Flüssigkristallanzeige, angezeigt und in einem nicht dargestellten Speicher des Geräts 1 abgelegt werden.

Der Speicher bildet zusammen mit einer Steuer- und Auswerteeinheit 8, beispielsweise einem Mikroprozessor, und der Anzeigeeinrichtung 7 eine elektronische Baugruppe, welche beispielsweise die Auswertung und Darstellung von Auswertungsergebnissen einer Serie von Messergebnissen ermöglicht, die zusammen mit Datum und Uhrzeit der zugrundeliegenden Messung in dem Speicher gespeichert werden können.

Das Analysehandgerät 1 enthält zusätzlich eine mechanische Baugruppe, die zumindest eine Transporteinrichtung 9 für Testelemente umfasst. Bei dem dargestellten Ausführungsbeispiel bewirkt die Transporteinrichtung 9 ein Aufwickeln des Trägerbands 3 auf eine Aufwickelspule der Kassette 2 und ein Abwickeln von einer Vorratsspule 10, also einen Bandtransport. Bei einem Analysehandgerät, das wie das dargestellte Ausführungsbeispiel zugleich ein Stechgerät ist, enthält die mechanische Baugruppe zusätzlich einen Stechantrieb 11, um Lanzetten 4 in eine Stechbewegung zu versetzen. Der Stechantrieb 11 ist genauso genommen eine weitere Transporteinrichtung, da eine Lanzette bei einem Stich in Stichrichtung bewegt bzw. Transportiert wird. Bevorzugt enthält die mechanische Baugruppe zusätzlich auch einen Elektromotor 12. Prinzipiell kann die von dem Elektromotor an die Transporteinrichtung 9 und den Stechantrieb 11 gelieferte Kraft aber auch von einem Benutzer aufgebracht werden.

Der Stechantrieb 11 enthält vorzugsweise einen mechanischen Energiespeicher, beispielsweise ein Federelement aus Kunststoff oder Metall, der bei Bedarf die für einen schnellen Lanzettenstich benötigte kinetische Energie freisetzt. Ein solcher mechanischer Energiespeicher kann vorteilhaft von dem Elektromotor 12 des Analysehandgeräts 1 aufgeladen werden, beispielsweise indem eine Feder gespannt wird. Der Stechantrieb 11 kann beispielsweise eine Stichbewegung in der in der WO 2008/08344 A1 beschriebenen Weise bewirken.

Das dargestellte Analysehandgerät 1 hat ein erstes Betätigungselement 13, durch dessen Betätigung das Gerät 1 in einen ersten aktiven Zustand versetzt, die mechanische Baugruppe aktiviert und eine Messung vorbereitet wird. Das erste Betätigungselement 13 ist bevorzugt als eine Abdeckung einer Geräteöffnung ausgebildet, an der eine Probenaufnahme erfolgt. Das erste Betätigungselement 13 wird also dadurch betätigt, dass es aus einer die Geräteöffnung abdeckenden Ausgangsposition in eine andere Position bewegt wird, in der die Geräteöffnung freigelegt ist.

Beispielsweise kann das erste Betätigungselement 13 schwenkbar oder verschiebbar an dem Gerätegehäuse 14 befestigt sein. Das erste Betätigungselement 13 kann beispielsweise als Schutzkappe ausgebildet sein und das Innere des Geräts 1 vor Verschmutzung schützen. Indem das erste Betätigungselement 13 als eine Abdeckung der Geräteöffnung ausgeführt ist, wird ein hoher Benutzerkomfort erreicht, da die mechanische Baugruppe nur dann aktiviert wird, wenn ein Benutzer auch tatsächlich eine Messung vornehmen möchte, da der Benutzer in der Regel nur dann die Gehäuseöffnung freilegen wird. Ein wichtiger Vorteil des als Abdeckung ausgebildeten Betätigungselements 13 ist dabei insbesondere auch, dass ein Benutzer die Zeit zwischen dem Bewegen der Abdeckung und dem Anlegen eines Fingers an die Gehäuseöffnung nicht als Wartezeit empfindet, dennoch aber zwischen dem Bewegen der Abdeckung und dem Anlegen eines Fingers an die Gehäuseöffnung in der Regel genug Zeit ist, um das Gerät auf eine Messung vorzubereiten. Ein Benutzer hat deshalb den Eindruck, dass eine Messung "sofort" gestartet werden kann.

Wenn ein Benutzer keine Messung durchführen möchte, sondern beispielsweise lediglich Geräteeinstellungen ändern oder sich vorhandene Messergebnisse anzeigen lassen möchte, kann er das Gerät 1 durch Betätigung eines zweiten Betätigungselements 15, das beispielsweise als eine Taste ausgebildet sein kann, in einen zweiten aktiven Zustand versetzen und die Anzeigeeinrichtung 7 ohne Aktiveren der mechanischen Baugruppe einschalten. Auf der die Anzeigeeinrichtung 7 tragenden Vorderseite des Handgeräts 1 sind bei dem dargestellten Ausführungsbeispiel weitere Betätigungselemente 16 angeordnet. Diese können beispielsweise verwendet werden, um Befehle aus einem von der Anzeigeeinrichtung 7 angezeigten Bedienmenü auszuwählen. Die Anzeigeeinrichtung 7 kann dabei als Sensorfeld ausgebildet sein und ein zusätzliches Betätigungselement bilden.

Wenn sich das Analysehandgerät 1 in dem ersten aktiven Zustand befindet, kann durch Betätigen eines seiner Betätigungselemente eine Messung gestartet werden, bei dem dargestellten Ausführungsbeispiel also ein Lanzettenstich ausgelöst werden. Bevorzugt ist hierfür ein drittes Betätigungselement 17 vorgesehen, das an der Geräteöffnung angeordnet ist. Das dritte Betätigungselement 17 wird durch Anlegen eines Körperteils, in dem eine Stichwunde erzeugt werden soll, an die Geräteöffnung betätigt. Bevorzugt enthält das dritte Betätigungselement 17 einen Temperatur- und/oder Drucksensor, der nicht nur das Anlegen eines Körperteils feststellt, sondern zusätzlich auch überprüft, ob für eine Probenentnahme günstige Bedingungen vorliegen, beispielsweise der Druck, mit dem das Körperteil auf der Geräteöffnung lastet, oder die Temperatur des Körperteils in einem vorgegebenen Bereich liegen.

Bevorzugt deckt das erste Betätigungselement 13 in seiner die Geräteöffnung abdeckenden Ausgangsposition das dritte Betätigungselement 17 ab, so dass dieses im inaktiven Zustand des Geräts 1 geschützt ist.

Nach Abschluss einer Messung geht das Analysehandgerät 1 selbsttätig, also ohne Zutun eines Benutzers, in den zweiten aktiven Zustand über, so dass ein Benutzer zusätzliche Gerätefunktionen nutzen, beispielsweise Auswertungen einer Serie von Messungen betrachten und so seinen Behandlungsfortschritt überprüfen, oder Geräteeinstellungen ändern kann, beispielsweise Weck- oder Erinnerungsfunktionen auf andere Uhrzeiten verstellen kann.

Der Elektromotor 12 und die elektronische Baugruppe mit der Anzeigeeinrichtung 7 und der Steuer- und Auswerteeinheit 8 werden von einer oder mehreren Batterien 18 gespeichert, die in ein dafür vorgesehenes Fach des Geräts 1 eingelegt und bei Bedarf ausgetauscht werden können.

### Bezugszeichen

- 1: Analysehandgerät
- 2: Magazin
- 3: Trägerband
- 4: Lanzetten
- 5: Testelemente
- 6: Sensor
- 7: Anzeigeeinrichtung
- 8: Steuer- und Auswerteeinheit
- 9: Transporteinrichtung
- 10: Vorratsspule
- 11: Stechantrieb
- 12: Elektromotor
- 13: Erstes Betätigungselement
- 14: Gerätegehäuse
- 15: Zweites Betätigungselement
- 16: Weitere Betätigungselemente
- 17: Drittes Betätigungselement
- 18: Batterien
- 20: Gehäusedeckel

## Patentansprüche

1. Analysehandgerät mit
einer mechanischen Baugruppe, die eine Transporteinrichtung (9) für analytische Verbrauchselemente (4, 5) umfasst,
einer Anzeigeeinrichtung (7),
einem Sensor (6) zur Untersuchung einer durch einen Lanzettenstich gewonnenen Körperflüssigkeitsprobe,
einem Speicher zum Speichern einer Serie von Messergebnissen, und Betätigungselementen (13, 15, 16, 17) zum Bedienen des Analysehandgeräts (1),
**gekennzeichnet durch**
ein erstes Betätigungselement (13), **durch** dessen Betätigung das Gerät (1) in einen ersten aktiven Zustand versetzt wird, die mechanische Baugruppe aktiviert und eine Messung vorbereitet wird, wobei in dem ersten aktiven Zustand **durch** Betätigen eines der Betätigungselemente (13, 15, 16, 17) eine Messung gestartet wird, und
einem zweiten Betätigungselement (15), **durch** dessen Betätigung das Gerät (1) in einen zweiten aktiven Zustand versetzt und die Anzeigeeinrichtung (7) ohne aktivieren der mechanischen Baugruppe eingeschaltet wird.

2. Analysehandgerät nach Anspruch 1, **dadurch gekennzeichnet, dass** das erste Betätigungselement (13) eine Abdeckung einer Geräteöffnung ist, an der eine Probenaufnahme erfolgt.

3. Analysehandgerät nach Anspruch 2, **dadurch gekennzeichnet, dass** die Abdeckung schwenkbar ist.

4. Analysehandgerät nach Anspruch 2 oder 3, **dadurch gekennzeichnet, dass** zum Auslösen einer Messung ein drittes Betätigungselement (17) vorgesehen ist, das an der Geräteöffnung angeordnet ist und durch Anlegen eines Körperteils, in dem eine Stichwunde erzeugt werden soll, an die Geräteöffnung betätigt wird.

5. Analysehandgerät nach Anspruch 4, **dadurch gekennzeichnet, dass** das erste Betätigungselement (13) in seiner die Geräteöffnung abdeckenden Ausgangsposition das dritte Betätigungselement (17) abdeckt.

6. Analysehandgerät nach einem der vorstehenden Ansprüche, **dadurch gekennzeichnet, dass** die mechanische Baugruppe einen Stechantrieb (11) umfasst, um Lanzetten (4) in eine Stechbewegung zu versetzen.

7. Analysehandgerät nach Anspruch 6, **dadurch gekennzeichnet, dass** eine Messung vorbereitet wird, indem ein mechanischer Energiespeicher des Stechantriebs (11) aufgeladen wird.

8. Analysehandgerät nach Anspruch 7, **dadurch gekennzeichnet, dass** eine Messung vorbereitet wird, indem der Stechantrieb (11) an eine Lanzette (4) ankoppelt.

9. Analysehandgerät nach einem der vorstehenden Ansprüche, **dadurch gekennzeichnet, dass** die Verbrauchselemente Testelemente (5) und/oder Lanzetten (4) sind.

10. Analysehandgerät nach einem der vorstehenden Ansprüche, **dadurch gekennzeichnet, dass** die mechanische Baugruppe einen Elektromotor (12) umfasst.

11. Analysehandgerät nach einem der vorstehenden Ansprüche, **dadurch gekennzeichnet, dass** eine Messung vorbereit wird, indem ein Verbrauchselement (4, 5) von der Transporteinrichtung (9) aus einem Verbrauchselementevorrat entnommen wird.

12. Analysehandgerät nach einem der vorstehenden Ansprüche, **dadurch gekennzeichnet, dass** durch Betätigen des ersten Betätigungselements (13) die Anzeigeeinrichtung (7) eingeschaltet wird.

13. Analysehandgerät nach einem der vorstehenden Ansprüche, **dadurch gekennzeichnet, dass** in dem zweiten aktiven Zustand Geräteeinstellungen geändert werden können.

14. Analysehandgerät nach einem der vorstehenden Ansprüche, **dadurch gekennzeichnet, dass** in dem zweiten aktiven Zustand Messergebnisse von der Anzeigeeinrichtung (7) angezeigt werden können.

15. System mit einem Analysehandgerät (1) nach einem der vorstehenden Ansprüche und einem in das Gerät einsetzbaren Magazin (2), das einen Verbrauchselementevorrat enthält.
